# EUROPEAN PATENT SPECIFICATION

(11) **EP 1 357 839 B1**
(45) Date of publication and mention of the grant of the patent: **19.07.2006**
(21) Application number: 02715566.2
(22) Date of filing: 25.01.2002
(51) Int. Cl.: A61B 10/00

(54) **MEDICAL SPATULA**
MEDIZINISCHER SPATEL
SPATULE MEDICALE

(30) Priority: 06.02.2001 GB 0102952
(43) Date of publication of application: 05.11.2003
(73) Proprietor: Platinum Feather Consultants Limited, Tetbury, Glouchestershire GL8 8DY (GB)
(72) Inventor: PEARCE, Harold, James, Tetbury, Gloucestershire GL8 8DY (GB)
(74) Representative: Harrison, Ivor Stanley
(86) International application number: PCT/GB2002/000316
(87) International publication number: WO 2002/062225

(56) References cited:
- WO-A-99/25251
- US-A- 3 774 590
- US-A- 4 027 658
- US-A- 4 641 662
- US-A- 5 191 899

## Description

This invention relates to spatulas for taking cell samples from the body, for example for histological examination, especially for use in taking samples for analysis in screening for cervical cancer, for examination or investigation of DNA and for screening for HIV and other micro-organisms which may be present in cell samples from within the body.

Spatulas for cervical cancer screening are conventionally made from wood and comprise elongate body portion and a head portion shaped to have a side lobe. In use, the spatula is inserted in the vagina so that the head portion contacts the cervix and the body portion is then rotated about its longitudinal axis so that the inner edge of the side lobe of the head portion scrapes around the exterior wall of the cervix, removing therefrom a sample of cells. Conventionally, the sample is then transferred to a glass slide to provide a "smear" for histological examination by microscope. However such examination is necessarily subjective and therefore potentially unreliable and, indeed, the technique has occasionally given rise to publicly-expressed concerns regarding the effectiveness of screening programmes in some hospitals. In any event, the act of transferring the sample to the slide involves subjecting the sample to shearing forces which can physically damage or distort the cells, making interpretation of results more difficult.

In an attempt to avoid the problems of manual analysis, apparatus for automated examination by so-called liquid base cytology has more recently been developed. In use, the sample from the spatula is added to a liquid to form a suspension of cells and it has been found that the results of the ensuing examination are far more consistently reliable than with manual examination. However, a disadvantage is that the wooden spatula tends to absorb the liquid, resulting in difficulty in obtaining complete transfer of the sample to the liquid and a consequential weak or dilute liquid-base sample for cytological analysis.

It is an object of the present invention to provide a spatula of the type described which can be used for obtaining cervical samples both for manual and automated analysis but which especially overcomes the problems of wooden spatulas when the samples are analysed by liquid base cytology.

Another object of the invention is to provide a spatula which can be used for collection of samples from other body sites, for example the mouth or the vagina, for analysis to determine the DNA or for screening purposes in connection with HIV or other viral or bacterial infections.

US-A-3774590 describes a device for collection of tissue fragments from the human uterus, the device being formed from a plastics material and having handle and blade portions joined by a structurally weakened neck portion defined by a transverse groove. The blade portion, with collected matter thereon, can be separated from the handle portion and dropped into a biopsy bottle. US-A-4641662 describes a disposable curette scraper removable mounted on an elongate handle, the scraper being separable from the handle for placement in a jar of fixative and replaceable with another scraper.

According to one aspect of the present invention, a spatula for taking cell samples for examination is formed from a non-absorbent plastics material and comprises a body portion and a head portion, with a frangible zone between the body and head portions, the frangible zone comprising a lateral groove or channel, characterised in that the groove is terminated inwardly of the edges of the spatula by end walls.

In spatulas according to the invention, the head portion can be broken away from the body portion by relative bending about a lateral axis in the frangible zone, the frangible zone nevertheless providing sufficient strength to resist fracture under rotational forces about the longitudinal axis of the spatula in use.

The surface of the spatula, which is conventionally of flat configuration, or at least of the head portion thereof, is preferably of stippled or satin-effect texture for better adhesion of cells during the taking of the sample. The surface of at least the head portion may be provided with means for scraping the body part from which cells are collected, to increase the number of cells collected, or to improve retention on the head of the spatula of cells collected. Such means may comprise one or more protrusions such as ridges, extending generally above the plane of the surface, or depressions formed in the spatula generally below the plane of the surface.

The frangible zone provides a weakness in terms of relative bending resistance between the head and body portions about a lateral axis in the frangible zone while not significantly compromising the rotational strength as between the head and body portions under twisting forces about the longitudinal axis of the spatula.

Preferably, the spatula includes a frangible zone comprising a lateral groove or channel on both sides of the spatula. Optionally, each groove is provided with one or more intermediately-located fillets or webs to moderate the weakness. Where a groove is formed on each side of the spatula, they preferably coincide on respective sides whereby the lateral axis of weakness passes through the bridging material forming a common base to and extending between the respective grooves. The fillets are preferably alternately-disposed in such respective grooves.

A suitable plastics material for formation of spatulas according to the invention is a polyolefin such as polypropylene. For functional and economic reasons, the thickness of the spatulas should be within the range 1.0 to 3.00 mm, and the groove or grooves should extend in depth to approximately two thirds of the thickness in aggregate, especially where they are coincident on respective sides. Should the grooves be disposed in offset relationship, their aggregate depth could be greater, for example up to 1.5 times the thickness of the material.

Spatulas according to the invention may have an enlarged tail portion at the end of the body portion remote from the head portion, shaped for example as an oval, and such tail portion may likewise be formed with a frangible zone between the tail and body portions..

Embodiments of the invention will now be described by way of example with reference to the accompanying drawings of which:
Figure 1 is a view showing the front of the head end and the rear of the foot end on a scale of approximately 1:1 of a spatula according to the present invention;
Figures 2 is a fragmentary view on an enlarged scale of the part II of the spatula shown in Figure 1 and
Figure 3 is a front view of another spatula according to the invention, on a scale of approximately 1.1.

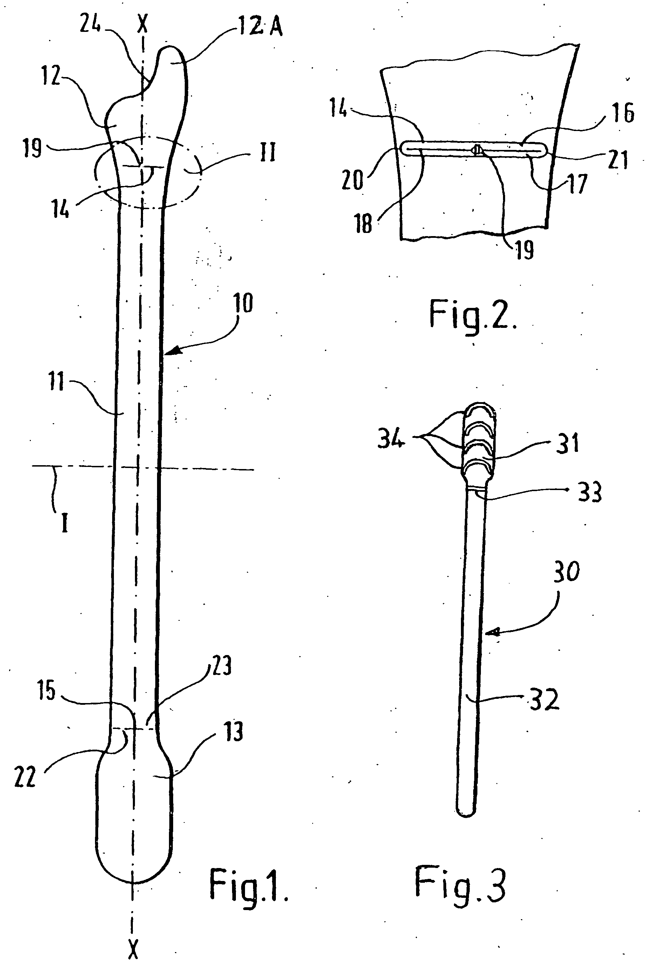

Referring first to Figure 1, the spatula shown generally 10 is formed from a flat strip of a thermoplastic plastics material having a slightly stippled or satin-effect surface and a thickness of 2 mm. The spatula consists of a body portion 11, a head portion 12 and a tail portion 13. For convenience in illustration, the front of the head portion and the upper part of the body portion is shown above the line I whereas the rear of the tail portion and the lower part of the body portion is shown below the line I, as though the spatula had been rotated about the X-X axis.

The head portion 12 has a generally flared profile with an axially-extending lobe 12a. The neck between the head and body portions is formed with a V-shaped groove 14, as described later with reference to and as shown in more detail in Figure 2. The tail portion 13 of the spatula is formed with an oval profile and also has a V-shaped groove 15.

Referring to Figure 2, the sides 16, 17 of the groove 14 slope inwardly to the bottom 18 and the groove is bridged centrally by a fillet 19 which extends almost to the front surface of the spatula. The groove terminates inwardly of the edges of the spatula in end walls 20, 21 and is this blind-ended.

Referring back to Figure 1, the groove 15 is similar in formation to the groove 14 but has two fillets 22, 23. A groove similar to groove 15 is also formed in the rear face of the spatula, immediately behind groove 14, and a groove similar to groove 14 is also formed in the front face of the spatula, immediately behind groove 15, whereby the fillets in each pair of grooves are in alternating disposition. Each groove is approximately one third the thickness of the spatula in depth and the material of the spatula between the bottoms of each respective back-to-back pair of grooves constitutes a frangible zone of weakness.

In use with the head end of the spatula inserted in the vagina, the inner edge 24 of the lobe 13 contacts the cervix and, with the spatula rotated about its longitudinal axis X-X, encircles the cervix and removes a sample of cells, the sample adhering to the surface of the head of the spatula adjacent the edge 24.

The sample cells may be smeared on a glass slide for conventional examination but for liquid base cytology they are required to be transferred to a liquid medium. For this purpose, the entire head end of the spatula is immersed in the liquid and, with the tip of the lobe 13 in contact with the base of the container, a downwards and sideways force is exerted on the body portion so that the head portion bends relative to the body portion about a lateral axis which coincides with the zone of weakness between the bottoms of the groove 14 and the corresponding groove formed in the rear face. The head may break off entirely from the body portion on being thus bent in one direction but preferably the frangible zone has sufficient strength, by virtue of the fillets to the grooves and the end walls of the grooves, to require bending in the other direction before complete fracture takes place. With such strength, the head portion will not break off under rotation force when in the vagina but will fracture cleanly on application of bending forces first in one direction and then in the other when in the sample liquid, whereby all the cell sample is transferred to the test liquid without physical damage.

With reference to Figure 3, the spatula shown generally at 30 is for use particularly for collection of oral cell samples by scraping the head of the spatula across the inner wall of the cheek, for example, but could be used for collection of cells from other body parts where a spatula as shown in Figures 1 and 2 would be inappropriate. The head 31 of the spatula is jointed to the body or handle part 32 by a neck which includes a groove 33 on one side and a corresponding groove (not shown) on the other side. The head on each side carries a series of arcuate ridge projections 34 which extend above the plane of the head and body or handle part and assist in removal and retention of cells pending the breaking off of the head in a liquid test medium, as already described.

The spatula is smaller than that described with reference to Figures 1 and 2 and the frangible zone defined by the groove 33 is not required to be as resistant to fracture under rotational forces; the end walls and intermediate fillets are therefore omitted in the embodiment illustrated but are optionally includable if necessary.

## Claims

1. A spatula (10, 32) for taking cell samples for histological examination, the spatula being formed from a non-absorbent plastics material and comprising a body portion (11) and a head portion (12) with a frangible zone between the body and head portions, the frangible zone comprising a lateral groove or channel (14), **characterised in that** the groove (14) is terminated inwardly of the edges of the spatula by end walls (20, 21).

2. A spatula according to claim 1, in which the surface is of stippled or satin-effect texture.

3. A spatula according to claim 1 or claim 2, in which the frangible zone comprises a lateral groove or channel (14) on both sides of the spatula.

4. A spatula according to any of claims 1 to 3, in which the or each groove (14) is provided with one or more fillets or webs (19) which are intermediately-located between the ends of the or each groove.

5. A spatula according to claim 4, in which a groove (14) is formed on each side and the fillets (19, 22, 23) are disposed at different positions along the length of the respective grooves.

6. A spatula according to any preceding claim, including an enlarged tail portion (13) at the end of the body portion (11) remote from the head portion (12) and formed with a frangible zone (15) between the tail and body portions.

7. A spatula according to any preceding claim, in which the head portion (12) includes means for scraping across the surface of a body part from which, in use, cells are to be collected.

8. A spatula according to claim 7, in which the scraping means comprise one or more protrusions (34) extending above the plane of the surface of the spatula (32).

## Patentansprüche

1. Spatel (10, 32) zur Entnahme von Zellproben für die histologische Untersuchung, wobei der Spatel aus einem nicht absorbierenden Kunststoffmaterial gebildet ist und einen Körperbereich (11) sowie einen Kopfbereich (12) aufweist, wobei sich zwischen dem Kopfbereich und dem Körperbereich eine Bruchzone befindet, wobei die Bruchzone eine in seitlicher Richtung verlaufende Nut oder einen in seitlicher Richtung verlaufenden Kanal aufweist,
**dadurch gekennzeichnet,**
**dass** die Nut (14) innerhalb von den Kanten des Spatels durch Endwände (20, 21) abgeschlossen ist.

2. Spatel nach Anspruch 1,
wobei die Oberfläche eine rasterartige Textur oder eine Textur mit Satin-Effekt aufweist.

3. Spatel nach Anspruch 1 oder 2,
wobei die Bruchzone eine in seitlicher Richtung verlaufende Nut oder einen in seitlicher Richtung verlaufenden Kanal (14) beidseits des Spatels aufweist.

4. Spatel nach einem der Ansprüche 1 bis 3,
wobei die oder jede Nut (14) mit einer oder mehreren Übergangsverbindungen oder Stegen (19) versehen ist, die zwischen den Enden der oder jeder Nut an zwischengeordneten Stellen angeordnet sind.

5. Spatel nach Anspruch 4,
wobei auf jeder Seite eine Nut (14) ausgebildet ist und wobei die Übergangsverbindungen (19, 22, 23) an unterschiedlichen Stellen entlang der Länge der jeweiligen Nuten angeordnet sind.

6. Spatel nach einem der vorausgehenden Ansprüche,
mit einem vergrößerten Endbereich (13), der an dem von dem Kopfbereich (12) abgelegenen Ende des Körperbereichs (11) vorgesehen ist und mit einer Bruchzone (15) zwischen dem Endbereich und dem Körperbereich ausgebildet ist.

7. Spatel nach einem der vorausgehenden Ansprüche,
wobei der Kopfbereich (12) Abstricheinrichtungen zum Streichen über die Oberfläche eines Körperteils beinhaltet, von dem im Gebrauch Zellen entnommen werden sollen.

8. Spatel nach Anspruch 7,
wobei die Abstricheinrichtungen einen oder mehrere Erhebungen (34) aufweisen, die über die Ebene der Oberfläche des Spatels (32) hinaus hochstehen.

## Revendications

1. Spatule médicale (10, 32) destinée au prélèvement d'échantillons de cellules en vue d'examen histologique, la spatule étant formée d'un matériau plastique non absorbant et comprenant une partie de corps (11) et une partie de tête (12) avec une zone cassable entre la partie de corps et la partie de tête, la zone cassable comprenant une rainure ou canal latéral (14), **caractérisée en ce que** la rainure (14) se termine à l'intérieur des bords de la spatule par des parois terminales (20, 21).

2. Spatule selon la revendication 1, dans laquelle la surface présente une texture en pointillés ou satinée.

3. Spatule selon la revendication 1 ou la revendication 2, dans laquelle la zone cassable comprend une rainure ou un canal latéral (14) sur les deux côtés de la spatule.

4. Spatule selon l'une quelconque des revendications 1 à 3, dans laquelle la ou chacune des rainures (14) est munie d'un ou plusieurs filets ou toiles (19) qui sont disposés de façon intermédiaire entre les extrémités de la ou de chacune des rainures.

5. Spatule selon la revendication 4, dans laquelle une rainure (14) est formée sur chaque côté et les filets (19, 22, 23) sont disposés à différents emplacements selon la longueur des rainures respectives.

6. Spatule selon l'une quelconque des revendications précédentes, comportant une partie terminale élargie (13) à l'extrémité de la partie de corps (11) à l'opposé de la partie de tête (12) et formée avec une zone cassable (15) entre la partie terminale et la partie de corps.

7. Spatule selon l'une quelconque des revendications précédentes, dans laquelle la partie de tête (12) comporte un moyen de raclage à travers la surface d'une partie de corps à partir de laquelle, pendant l'utilisation, des cellules doivent être recueillies.

8. Spatule selon la revendication 7, dans laquelle le moyen de raclage comprend une ou plusieurs saillies (34) s'étendant au dessus du plan de la surface de la spatule (32).
